# EUROPEAN PATENT APPLICATION

(11) **EP 4 588 490 A1**
(43) Date of publication of application: **23.07.2025**
(21) Application number: 24152780.3
(22) Date of filing: 19.01.2024
(51) Int. Cl.: A61K 49/00

(54) **DIAGNOSTIC USE OF IMMUNOGLOBULIN E**

(71) Applicant: Englmeier, Ludwig, 83080 Oberaudorf (DE)
(72) Inventor: Englmeier, Ludwig, 83080 Oberaudorf (DE)
(74) Representative: Bobbert & Partner Patentanwälte PartmbB

(57) **Abstract**

The present disclosure relates to the use of IgE for identifying individuals with genetic defects that lead to the expression of protein truncations, extended proteins, to a loss of protein expression or to the expression of mutated proteins. The method is based on a skin prick test, wherein IgE that is specific for the protein of interest is administered onto the skin. If the protein or protein variant that is specifically identified by IgE is present in the skin, the IgE, which was allowed to enter into the skin by the skin prick, will bind the target protein and then the protein-bound IgE will trigger mast cells and/or basophiles in the skin to release granules, resulting in the development of an erythema.

## Description

### BACKGROUND OF THE INVENTION

Genetic defects are typically identified by expensive methods which are based on either sequencing, blood analytics or biochemical assays. One can take cystic fibrosis, the most common hereditary disease which is caused by deleterious mutations of the CFTR gene, as just one example. While the disease was conventionally diagnosed in several phases based on observations, sequencing based methods for the identification of affected individuals are now used. Nunziato et al. (1) suggest to sequence DNA obtained from Guthrie spots in order to identify CFTR mutations by next generation sequencing molecular analysis shortly after birth. Spinal muscular atrophy, another frequent hereditary disease, is often caused by a deletion of exon7 of the SMN1 gene and - in particular in countries which cannot afford sequencing-based newborn screening - is often diagnosed only once the first symptoms have become manifest. However, the latest treatments, like onasemnogene abeparvovec therapy, need to be started as soon as possible after birth for best efficacy (2). Also in this case, the DNA-based methods which are employed for diagnosis in high-income countries are not generally available in many countries.

Poorer countries often do not have access to the expensive analytical machinery that is being used in countries with a well-funded healthcare system. There is a need for a simple and inexpensive method for identifying pathogenic protein variants - be it truncations, loss of protein expression, extensions (like polyglutamine tracts) or mutations.

### SUMMARY OF THE INVENTION

This problem is solved by the present invention, in particular, by the subject-matter of the claims. The present invention provides IgE for use in a diagnostic method, for example practiced on the human or animal body, e.g. a method comprising administering IgE to a subject in a skin-prick test. This can advantageously be used for detecting the presence or absence of a protein or a protein variant, e.g., for identifying individuals with genetic defects that lead to the expression of protein truncations, extended proteins, to a loss of protein expression or to the expression of mutated proteins. The method is preferably based on a skin prick test, wherein IgE that is specific for the protein of interest is administered onto the skin. The protein to which the IgE can specifically bind can, for example, exist in at least one pathogenic and at least one non-pathogenic variant. If the protein or protein variant that is specifically identified by IgE is present in the skin, the IgE, which is allowed to enter into the skin by the skin prick, will bind the target protein and then the protein-bound IgE will trigger mast cells and/or basophiles in the skin to release granules, resulting in the development of an erythema.

The rationale behind this assay is based on a reversal of the logic of the well-known allergy tests, where antigens, such as allergens, are administered into the skin by a skin prick and the presence or absence of antigen-specific IgE in the skin leads to either the development of an erythema (in the case that antigen-specific IgE is present) or to no response (if antigen-specific IgE is absent in the skin). In the present invention, this logic is reversed: it is the IgE that is supplied via the skin-prick, not the allergen. And it is the presence or absence of the protein (for which the administered IgE is specific) within the skin that is detected, not the presence or absence of antigen-specific IgE. Without wishing to be bound by theory, the skin prick serves at least two functions: it allows IgE to enter the skin and it destroys some of the cells of the skin, thereby making also intracellular proteins of interest accessible to the added IgE.

The assay uses low amounts of IgE and is therefore considered safe, and its simplicity allows not only for the detection of pathogenic protein variants in individuals which are suspected to carry such a pathogenic protein variant, for example because they already show some symptoms of the disorder which is typically caused by said pathogenic protein variant, but the assay also enables the screening of large numbers of unsymptomatic individuals at low cost so as to identify those who do express a pathogenic protein variant regardless of any particular suspicion that they have the disease.

Moreover, by using a collection of IgEs with different specificities as separate entities, for example as separate drops on the skin, the presence of several different pathogenic protein variants of interest can be assayed in one go, for example by applying different samples with different individual specific IgEs as separate drops to, for example, a forearm, as a collection of individual prick tests. This is a similar setting to the widely used allergy tests, where several different allergens are tested in one go, simply by applying separate drops containing individual allergens.

The invention therefore relates to IgE for use as a diagnostic agent. The invention also relates to IgE for use for use in a method of diagnosis, for example practiced on the human or animal body, such as practiced on the skin of the human or animal body. An example of such a method is a skin-prick test. If IgE that is capable of binding specifically to a pathogenic variant of a protein, but not to the non-pathogenic version of said protein is used, the development of an erythema is indicative of the presence of a pathogenic variant of said protein. If IgE that is capable of binding specifically to the non-pathogenic version of a protein, but not to a pathogenic variant of said protein is used (for example an IgE that binds to the C-terminus of the non-pathogenic full-length protein, which is absent in a pathogenic truncated variant of said protein), then the absence of the development of an erythema is indicative of the absence of the non-pathogenic version, which allows for the conclusion that the specific biochemical functionality provided by the non-pathogenic version of said protein might be missing or compromised, for example because only a pathogenic variant of said protein is expressed or because said protein is not expressed at all. In particular monogenic disorders can be identified by the IgE-based diagnosis or screened for by the IgE-based diagnostic method of the invention. Examples are a neurodegenerative disorder, such as spinal muscular atrophy, or a primary immunodeficiency, such as IgA deficiency. For example, IgE which is capable of binding a protein encoded by disease genes, such as encoded by, for example, CFTR, SMN1, DMD, LDLR, BRCA1, BRCA2, HTT, FMR1, ATXN1, ATXN2, ATXN3, TP53, SERPINA1, GALT, HFE, ATP7B, PKD1, PKD2 and NF1, to name but a few, is useful as a diagnostic agent of the invention.

The invention also relates to the use of IgE as a companion diagnostic for a medicament, in particular wherein the medicament is for the treatment of a genetic disorder. The invention also relates to a diagnostic method for detecting the presence of a pathogenic variant of a protein in the skin wherein,
a) IgE that is capable of binding a pathogenic variant of said protein, but not the non-pathogenic version of said protein, is applied to a site on the skin;
b) the skin is pricked at said site to allow for transfer of IgE into the skin, e.g. to allow for transfer of the IgE at least through the stratum corneum and stratum lucidum; and
c) wherein the development of an erythema is indicative of the presence of a pathogenic variant of said protein.

This method of detecting a pathogenic variant of the protein is also useful, when the individual is heterozygous for the non-pathogenic version of the protein and a pathogenic variant of the protein which acts as a dominant allele.

The invention also relates to a diagnostic method for detecting the presence of a pathogenic variant of a protein in the skin wherein,
a) IgE that is capable of binding the non-pathogenic version of a protein, but not a pathogenic variant of said protein, is applied to a site on the skin;
b) the skin is pricked at said site to allow for transfer of IgE into the skin, e.g. to allow for transfer of the IgE at least through the stratum corneum and stratum lucidum; and
c) the absence of the development of an erythema is indicative of the absence of the non-pathogenic version of said protein.

### REFERENCES

1. Nunziato M, Starnone F, Giordano S, D'Antonio M, Scognamiglio D, Esposito MV, et al. One-step NGS molecular analysis of the CFTR gene on newborn dried blood spots gives a higher diagnostic sensitivity in affected and carrier subjects: A pilot study. Clin Chim Acta. 2023;552:117625.
2. Sawada T, Kido J, Yae Y, Yuge K, Nomura K, Okada K, et al. Gene therapy for spinal muscular atrophy is considerably effective when administered as early as possible after birth. Mol Genet Metab Rep. 2023;35:100973.
3. Brooks E, Arrasate M, Cheung K, Finkbeiner SM. Using antibodies to analyze polyglutamine stretches. Methods Mol Biol. 2004;277:103-28.
4. Kandari D, Bhatnagar R. Antibody engineering and its therapeutic applications. Int Rev Immunol. 2023;42(2):156-83.
5. Wang B, Gallolu Kankanamalage S, Dong J, Liu Y. Optimization of therapeutic antibodies. Antib Ther. 2021;4(1):45-54.
6. Kim J, McFee M, Fang Q, Abdin O, Kim PM. Computational and artificial intelligence-based methods for antibody development. Trends Pharmacol Sci. 2023;44(3):175-89.
7. Mullard A. FDA approves 100th monoclonal antibody product. Nat Rev Drug Discov. 2021;20(7):491-5.
8. Ward ES, Güssow D, Griffiths AD, Jones PT, Winter G. Binding activities of a repertoire of single immunoglobulin variable domains secreted from Escherichia coli. Nature. 1989;341(6242):544-6.
9. Bird RE, Hardman KD, Jacobson JW, Johnson S, Kaufman BM, Lee SM, et al. Single-chain antigen-binding proteins. Science. 1988;242(4877):423-6.
10. Metzger H. The receptor with high affinity for IgE. Immunol Rev. 1992;125:37-48.
11. Knappik A, Ge L, Honegger A, Pack P, Fischer M, Wellnhofer G, et al. Fully synthetic human combinatorial antibody libraries (HuCAL) based on modular consensus frameworks and CDRs randomized with trinucleotides. J Mol Biol. 2000;296(1):57-86.
12. Apgar TL, Sanders CR. Compendium of causative genes and their encoded proteins for common monogenic disorders. Protein Sci. 2022;31(1):75-91.

### DETAILED DESCRIPTION OF THE INVENTION

The present invention relates to the first diagnostic use of IgE, in particular wherein IgE is used in the context of a skin-prick test. The present invention provides IgE for use in a diagnostic method, for example practiced on the human or animal body, e.g. a method comprising administering IgE to a subject in a skin-prick test.

The invention relates to a method where IgE is used for detecting the presence of a protein of interest, such as pathogenic variant of a protein of interest, in the skin. An IgE that is capable of binding to said protein of interest, such as said pathogenic variant of the protein of interest (but not to the non-pathogenic version of said protein), is applied to the skin (e.g. topically), for example as a drop that is layered onto the skin, such as onto the skin of the inner forearm. The skin is then pricked, for example gently with an allergy test lancet so that IgE can enter the skin. If the protein of interest, such as the pathogenic protein variant of interest, that is specifically bound by IgE is present in the skin, the IgE which was added by the skin prick binds the target protein and then the protein-bound IgE triggers mast cells and/or basophiles in the skin to release granules, resulting in the development of a skin reaction, such as an allergic skin reaction, for example including erythema. The development of a skin reaction, such as an allergic skin reaction, for example the development of erythema, is then indicative of the presence of said protein of interest in the skin, for example indicative for the presence of a pathogenic variant of said protein of interest. The skin reaction typically develops quickly, for example within 5-90 minutes, e.g., 15-60 minutes of the skin prick. The skin can be cleaned before the application of the IgE, e.g., using an alcohol such as ethanol.

In the above-described method, the absence of the skin reaction is then indicative of the absence of the pathogenic variant of said protein of interest.

The logic of this test can also be reversed. The disclosure therefore also relates to IgE for use in a diagnostic method where IgE is used for detecting the absence of a protein of interest, such as for exmple the absence of the wildtype version of a protein of interest, in the skin. An IgE that is capable of binding to said wildtype version of the protein of interest (but not to the pathogenic version of said protein), is applied to the skin, for example as a drop that is layered onto the skin, such as onto the skin of the inner forearm. The skin is then pricked, for example gently with an allergy test lancet, so that the IgE can enter the skin. If the wildtype version of the protein of interest that is specifically bound by IgE is present in the skin, the IgE, which was added by the skin prick binds the target protein and then the protein-bound IgE will trigger mast cells and/or basophiles in the skin to release granules, resulting in the development of a skin reaction, such as an allergic skin reaction, for example including erythema. The development of a skin reaction, such as an allergic skin reaction, for example the development of erythema, is then indicative of the presence of said wildtype version of the protein of interest in the skin. Conversely, the absence of the skin reaction is then indicative of the absence of the wildtype version of said protein of interest and might then indicate the presence of a disorder, for example a pathogenic state, a predisposition to develop a pathogenic state and/or a vulnerability towards an infection. The skin reaction typically develops quickly, for example within 5-90 minutes, e.g., 15-60 minutes of the skin prick. The skin can be cleaned before the application of the IgE, e.g., using an alcohol such as ethanol.

The skilled person will understand that the detection of a pathogenic protein variant by the method of the invention in an individual means that this individual is diagnosed as having a disorder or having a predisposition (a medical susceptibility) to develop a disorder which is associated with said pathogenic protein variant. This, whenever a method of detecting a pathogenic protein variant is disclosed, a method of diagnosing a predisposition for developing a disorder which is associated with said pathogenic protein variant is disclosed as well. Depending on the disorder, the presence of the disorder itself can also be diagnosed.

The IgE for diagnostic use, for example in the context of a diagnostic method practiced on the human or animal body, in particular on the human body, such as the two methods described just above, is preferably specific for a protein of interest. For example, the IgE may specifically bind to a pathogenic variant of a protein, but not the non-pathogenic version of said protein, or the IgE may specifically bind the non-pathogenic version of a protein, but not to a pathogenic variant of said protein. In the first case the IgE is specific for (a) pathogenic variant(s) of interest of a protein of interest. In the second case the IgE is specific for (a) non-pathogenic version(s) of a protein of interest, such as specific for the wildtype version of said protein of interest.

A pathogenic protein variant typically has a structural change compared to the wildtype version of said protein. Such a structural change can be the addition of amino acids to the protein of interest, for example at the N-terminus, within the protein sequence or at the C-terminus. Examples of such pathogenic protein variants are proteins with extended polyglutamine stretches, such as with more than 30 consecutive glutamines, for example with 35 to 200 consecutive glutamines.

Further examples of structural differences compared to the wildtype version of a protein that can lead to a pathogenic protein variant are truncations (where one or more amino acids at the C-terminus of a protein are missing), deletions (for example where one or more amino acids within the polypetide sequence of the wildtype version of a protein are missing), point mutations, changes in covalent protein modifications, such as changes in the phosphorylation, glycosylation, lipidation, ubiquitination, disulfide formation, cleavage of a peptide bond and carbonylation of a protein. Such changes can occur even if the wildtype polypeptide sequence of a protein of interest is intact, for example as a consequence of the mutation in another protein that mediates phosphorylation, dephosphorylation, glycosylation, deglycosylation, lipidation, delipidation, ubiquitination, deubiquitination, disulfide formation, disulfide reduction, cleavage of a peptide bond, carbonylation or decarbonylation of other proteins. The person skilled in the art will understand that such proteins are preferred where the wildtype version of the protein has at least low protein expression in at least one cell type present in the skin. Accordingly, the protein can be bound by the IgE and can form protein-IgE complexes that can activate mast cells in the skin. The reference for the level of protein expression in the skin is the Human Protein Atlas (www.proteinatlas.org) as of January 1^{st}, 2024. More preferably the wildtype version of the protein has at least medium protein expression in at least one cell type present in the skin. Preferably, the cell type present in the skin where the protein of interest is expressed is a fibroblast, an epidermal cell, a keratinocyte, a lymphocyte, a Langerhans cell and/or a melanocyte.

Non-limiting examples of proteins, for which pathogenic protein variants leading to disorders are known, are CFTR, SMN1, DMD, LDLR, BRCA1, BRCA2, HTT, FMR1, ATXN1, ATXN2, ATXN3, TP53, SERPINA1, GALT, HFE, ATP7B, PKD1, PKD2 and NF1 to name but a few. This list comprises examples of monogenic disorders, for example examples of protein involved in polyglutamine disease (such as HTT, FMR1, ATXN1, ATXN2 and ATXN3). This list also comprises examples of proteins involved in neurodegenerative disease (such as SMN1, DMD, HTT, ATXN1, ATXN2 and ATXN3).

Another non-limiting example of disorders where the use of IgE for diagnosis can be valuable are inborn errors of immunity, for example primary immunodeficiencies such as common variable immunodeficiency. There are more than 200 different forms of primary immunodeficiencies.

Non-limiting examples are BENTA, Immunodeficiency 11A and Immunodeficincy 11B, caused by pathogenic variants of CARD11. The present disclosure relates to the diagnostic uses described above of IgE which specifically binds CARD11, and in particular the diagnostic use of IgE which binds specifically a pathogenic variant of CARD11, for example any one of the pathogenic variants listed in UniProt entry Q9BXL7.

A non-limiting example is caspase 8 deficiency, caused by pathogenic variants of CASP8. The present disclosure relates to the diagnostic uses described above of IgE which specifically binds CASP8, and in particular the diagnostic use of IgE which binds specifically a pathogenic variant of CASP8, for example any one of the pathogenic variants listed in UniProt entry Q14790.

A non-limiting example is immunodeficiency 103, caused by pathogenic variants of CARD9. The present disclosure relates to the diagnostic uses described above of IgE which specifically binds CARD9, and in particular the diagnostic use of IgE which binds specifically a pathogenic variant of CARD9, for example any one of the pathogenic variants listed in UniProt entry Q9H257.

A non-limiting example is CTLA4 deficiency, caused by pathogenic variants of CTLA4. The present disclosure relates to the diagnostic uses described above of IgE which specifically binds CTLA4, and in particular the diagnostic use of IgE which binds specifically a pathogenic variant of CTLA4, for example any one of the pathogenic variants listed in UniProt entry P16410.

Another non-limiting example of disorders where the use of IgE for diagnosis can be valuable are autoimmune disorders such as systemic lupus erythematosus and rheumatoid arthritis.

The skilled person is well aware of the procedures for how an antibody with a desired specificity and having a desired binding specificity for the high affinity receptor for IgE (FcεRI) can be generated.

Since antibodies are modular proteins where the antigen binding site and the Fc-receptor binding site are separated, an IgE with a desired binding site can be generated from an existing antibody of another immunoglobulin isotype, such as an existing IgG or IgA antibody with a desired binding specificity, and then replacing the Fc part of these IgG or IgA antibodies with the Fc-part of human IgE, for example by exchanging the CH2 and CH3 domains of the heavy chain of these existing IgG or IgA antibodies for the three Ch2, CH3 and CH4 domains of the IgE heavy chain. In this way existing antibodies like, for example, a monoclonal human antibody to polyglutamine repeats, like the monoclonal antibody derived from hybridoma 3B5H10 and available from Absolute Antibody (further polyglutamine specific antibodies are described in (3)) can be class switched. The skilled person can determine the CDR of said antibody and then, equipped with this information, construct an antibody having the same CDR, but an Fc part of human IgE.

IgE are preferably human antibodies for use in a human subject but they can also be humanized or chimeric antibodies. Antibodies from other species, e.g., mouse, can also be used, e.g., for diagnosis o the presence or absence of a pathogenic variant of a protein in a mouse.

Antibodies with a complementary determining region having pretty much any desired antigen binding specificity can be obtained by traditional antibody engineering technologies, which are by now well established, robust and well within the skill of the person skilled in the art, as described in (4). Optimization of these antibodies is by now also well established (5). The field is now so advanced that Artificial Intelligence methods are used for antibody development, that computational predictors of antibody properties and structure are routinely employed and that computational antibody design methods are employed based on various machine learning models. In particular the design of complementarity-determining region loops can be done based on computer calculations (reviewed in (6)). With more than 100 FDA approved antibodies it is also clear that recombinant antibody production is so well established that it does not need to be explained any further (7).

The disclosure also broadly relates to the use of IgE in a skin-prick test. For example, IgE can be used as a companion diagnostic for a medicament. As a non-limiting example, onasemnogene abeparvovec is now being used for the treatment of spinal muscular atrophy (SMA), which can be caused by pathogenic variants of SMN1. An IgE which is specific for SMN1 can be used as a companion diagnostic for onasemnogene abeparvovec. Thus, the disclosure relates to the use of IgE as a companion diagnostic for a medicament, wherein the medicament is for the treatment of a genetic disorder, in particular wherein the genetic disorder is monogenic.

Thus, the disclosure relates to the use of IgE as a companion diagnostic for a medicament, wherein the medicament is for the treatment of a genetic disorder and wherein the genetic disorder is autosomal dominant, autosomal recessive or X-linked.

Therefore the invention relates to a method of treating a subject having a genetic disorder, such as an autosomal dominant, autosomal recessive or X-linked disorder, comprising administering an effective amount of a medicament effective for the treatment of said disorder to said subject, wherein the disorder has been diagnosed using a method of diagnosis of the invention.

For example, the invention relates to a method of treating a subject having spinal muscular atrophy, comprising administering an effective amount of a survival of motor neuron 2-directed RNA splicing modifier or comprising administering an effective amount of a gene therapy, such as an adeno-associated virus (AAV) vector-based gene therapy, to said subject, wherein SMA has been diagnosed using a method of diagnosis of the invention.

Stable aqueous formulations of antibodies are by now well known in the art, for example as described in WO2013/166448A1, US8906371B2 or WO2011/050242A1 to name just few examples. The present disclosure therefore also relates to an aqueous solution comprising IgE for diagnostic use.

### Definitions

The term "gene" means a DNA sequence that codes for an RNA or a particular sequence of amino acids which comprise all or part of one or more proteins or enzymes, and may or may not include regulatory DNA sequences, such as promoter sequences, which determine for example the conditions under which the gene is expressed. Herein genes are identified by their human gene symbol as defined by the HUGO Gene nomenclature committee. The relevant date is November 21, 2023.

A "promoter" or "promoter sequence" is a DNA regulatory region capable of binding RNA polymerase in a cell and initiating transcription of a downstream (3' direction) coding sequence. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

Within the context of the present invention the terms "mutant" and "mutation" mean a detectable change in genetic material, i.e. genomic DNA. Mutations include deletion, insertion or substitution of one or more nucleotides. The mutation may occur in the coding region of a gene (i.e. in exons), in introns, or in the regulatory regions (e.g. enhancers, response elements, suppressors, signal sequences, polyadenylation sequences, promoters) of the gene. Generally, a mutation is identified in a subject by comparing the sequence of a nucleic acid or polypeptide expressed by said subject with the corresponding nucleic acid or polypeptide expressed in a control population. Where the mutation is within the gene coding sequence, the mutation may be a "missense" mutation, where it replaces one amino acid with another in the gene product, or a "nonsense" mutation, where it replaces an amino acid codon with a stop codon. A mutation may also occur in a splicing site where it creates or destroys signals for exon-intron splicing and thereby lead to a gene product of altered structure. Within the context of the present invention a mutation is not silent, i.e. it results at least in an alteration of the amino acid sequence (where the gene product is a protein).

As used herein the term "deletion" means that a part of a protein-sequence is missing compared to a reference sequence for said protein.

As used herein the term "extension" means that a string of amino acids has been added to a protein-sequence when compared to a reference sequence for said protein.

The term "IgE" as used herein is used broadly and refers to a protein a) that comprises a region that is able to specifically bind to an antigen of interest, and b) that comprises a region that is able to bind to the high affinity receptor for immunoglobulin epsilon, FcεRI. Examples of "IgE" as used herein are antibodies and antibody fragments fused to an FcεRI binding region, and in particular bona fide IgE antibodies. The two regions a) and b) are spatially separated, as exemplified in an IgE antibody.

The term "antibody" as used herein refers to a protein comprising at least two heavy (H) chains and two light (L) chains inter-connected by disulfide bonds. An antibody can interact with an antigen. Each heavy chain is comprised of a heavy chain variable region or domain (abbreviated herein as VH) and a heavy chain constant region. The heavy chain constant region is comprised of three domains, CH1, CH2 and CH3, and in the case of an IgE antibody a fourth domain CH4. Each light chain is comprised of a light chain variable region or domain (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FR's arranged from amino- terminus to carboxy-terminus in the following order: FR1, CDR1, FR2, CDR2, FR3, CDR3, and FR4. The variable regions of the heavy and light chains contain a binding domain that interacts with an antigen. The constant region of the antibody mediates the binding of the immunoglobulin to the high affinity receptor for immunoglobulin epsilon. The term "antibody" includes for example, monoclonal antibodies, human antibodies, humanized antibodies, camelised antibodies and chimeric antibodies.

The phrase "antibody fragment", as used herein, refers to one or more portions of an antibody that are able to specifically bind an antigen. Examples of binding fragments include, but are not limited to, a Fab fragment, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (8), which consists of a VH domain; and an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv) (9). Such single chain antibodies are also intended to be encompassed within the term "antibody fragment". These antibody fragments are obtained using conventional techniques known to those of skill in the art, and the fragments are screened for utility in the same manner as are intact antibodies.

An example of a region that is able to bind to the high affinity receptor for immunoglobulin epsilon is the constant region of the human IgE antibody which binds the FcεRI with an affinity of 10¹⁰ M⁻¹ (10).

A "human antibody" or "human antibody fragment", as used herein, includes antibodies and antibody fragments having variable regions in which both the framework and CDR regions are derived from sequences of human origin. Furthermore, if the antibody contains a constant region, the constant region also is derived from such sequences. Human origin includes, e.g., human germline sequences, or mutated versions of human germline sequences or antibody containing consensus framework sequences derived from human framework sequences analysis, for example, as described in (11).

A "humanized antibody" or "humanized antibody fragment" is defined herein as an antibody or antibody fragment which has constant antibody regions derived from sequences of human origin and the variable antibody regions or parts thereof or only the CDRs are derived from another species. For example, a humanized antibody can be CDR-grafted, wherein the CDRs of the variable domain are from a non-human origin, while one or more frameworks of the variable domain are of human origin and the constant domain (if any) is of human origin.

As used herein the term "expression" refers to gene expression of a polypeptide or protein.

As used herein the "expression of an mRNA" relates to the transcriptional level of gene expression.

As used herein an "exon" is any part of a gene that will encode a part of the final mature RNA produced by that gene after introns have been removed by RNA splicing. The term exon refers to both the DNA sequence within a gene and to the corresponding sequence in RNA transcripts. In RNA splicing, introns are removed and exons are covalently joined to one another as part of generating the mature messenger RNA.

"Population" means a group of individual organisms of the same species that live in the same geographic area. For example, all human beings living in the United States of America form the "population" of the USA. Or all cows of a farm form the population of cows on the farm. "Subpopulation" means a proper subset of the population, wherein the individuals from the subset share at least one characteristic with one another that is not shared by all individuals of the population. For example, old people above the age of 60 in the USA would form a subpopulation within the larger population of the USA.

The "non-pathogenic version of a protein" is the mature form of the protein derived from the reference protein sequence listed in UniProtKB for said protein. For example, for TLR7 the UniProtKB is Q9NKY1. This entry provides a sequence for the 1049 amino acid TLR7 polypeptide. This is the reference sequence, and the mature form derived from this TLR7 after posttranslational modifications and processing is the "non-pathogenic version" of TLR7.

A "pathogenic variant" of a protein is a variant of a protein that is associated with a disease. Pathogenic variants are, for example, the variants listed as "likely pathogenic or pathogenic" or with "predicted consequences" in the UniProtKB database. Taking TLR7 as an example, the following is a non-exhaustive list of variants which are examples of a "pathogenic variant" of TLR7: VAR_087534, where Arginine at position 28 is changed to a glycine, VAR_087535: Y changed to H at position 264, RCV001254821: Q to missing at position 710, VAR_084629: V to F at position 795, or the various variants listed as having premature stop codons.

A genetic "predisposition" or "susceptibility" means that an individual who is predisposed or susceptible has a higher probability of developing a certain disease.

TLR7 as used herein relates, depending on the context, to the TLR7 gene or to the TLR7 protein, an endosomal receptor that plays a key role in innate and adaptive immunity (PubMed:14976261, PubMed:32433612). It is envolved in host immune response against pathogens through recognition of uridine-containing single strand RNAs (ssRNAs) of viral origin or guanosine analogs (PubMed:31608988, PubMed:27742543, PubMed:12738885, PubMed:32706371). Upon binding to agonists, the TLR7 receptor undergoes dimerization that brings TIR domains from the two molecules into direct contact, leading to the recruitment of the TIR-containing downstream adapter MYD88 through homotypic interaction (PubMed:27742543). In turn, the Myddosome signaling complex is formed involving IRAK4, IRAK1, TRAF6, TRAF3 leading to activation of downstream transcription factors NF-kappa-B and IRF7 to induce proinflammatory cytokines and interferons, respectively (PubMed:27742543, PubMed:32706371). TLR7 is described in detail as gene ID 51284 in the NCBI Gene database and is located on the X chromosome. The TLR7 protein has the UniProtKB ID Q9NYK1.

NFkB or NFkappaB as used herein relates, depending on the context, to the NFKB1 gene or its expression product. NFKB1 is described in detail as gene ID 4790 in the NCBI Gene database. The NFKB1 protein has the UniProtKB ID P19838. Two randomly selected examples of pathogenic variants are RCV001027589 and RCV000195130.

CFTR as used herein relates, depending on the context, to the CFTR gene or its expression product. CFTR is described in detail as gene ID 1080 in the NCBI Gene database. The CFTR protein has the UniProtKB ID P13569. The most common example of a pathogenic variant is VAR_000171 where the phenylalanine at position 508 is missing and CFTR is degraded. An example of a disease associated with a pathogenic variant of CFTR is cystic fibrosis.

SMN1 as used herein relates, depending on the context, to the SMN1 gene or its expression product. SMN1 is described in detail as gene ID 6606 in the NCBI Gene database. The SMN1 protein has the UniProtKB ID Q16637. According to the human protein atlas the SMN1 protein is expressed at medium levels in several cell types present in the skin. One example of a pathogenic variant is VAR_005617 where the tyrosine at position 272 is changed to a cysteine. An example of a disease associated with a pathogenic variant of SMN1 is spinal muscular atrophy.

DMD as used herein relates, depending on the context, to the DMD gene or its expression product. DMD is described in detail as gene ID 1756 in the NCBI Gene database. The DMD protein has the UniProtKB ID P11532. According to the human protein atlas the DMD protein is expressed at low levels in endothelial cells present in the skin. One example of a pathogenic variant is VAR_023547 where the cystein at position 3340 is changed to a tyrosin. An example of a disease associated with a pathogenic variant of DMD is duchenne muscular atrophy.

LDLR as used herein relates, depending on the context, to the LDLR gene or its expression product. LDLR is described in detail as gene ID 3949 in the NCBI Gene database. The LDLR protein has the UniProtKB ID P01130. One example of a pathogenic variant is VAR_005305 where the two amino acids at position 47 and 48 are missing. An example of a disease associated with a pathogenic variant of LDLR is familial hypercholesterolemia.

ATXN1 as used herein relates, depending on the context, to the ATXN1 gene or its expression product. ATXN1 is described in detail as gene ID 6310 in the NCBI Gene database. The ATXN1 protein has the UniProtKB ID P54253. According to the human protein atlas the ATXN1 protein is expressed at medium levels in epidermal cells and keratinocytes present in the skin. Examples of a pathogenic variant of ATXN1 are variants with a polyglutamine tract of 40 to 90 residues. An example of a disease associated with a pathogenic variant of ATXN1 is spinocerebellar ataxia 1.

ATXN2 as used herein relates, depending on the context, to the ATXN2 gene or its expression product. ATXN2 is described in detail as gene ID 6311 in the NCBI Gene database. The ATXN2 protein has the UniProtKB ID Q99700. According to the human protein atlas the ATXN2 protein is expressed at low levels in the epidermal cells present in the skin. Example of a pathogenic variant of ATXN2 are variants with a polyglutamine tract of 32 to 200 residues. An example of a disease associated with a pathogenic variant of ATXN2 is spinocerebellar ataxia 2.

ATXN3 as used herein relates, depending on the context, to the ATXN3 gene or its expression product. ATXN3 is described in detail as gene ID 4287 in the NCBI Gene database. The ATXN3 protein has the UniProtKB ID P54252. According to the human protein atlas the ATXN3 protein is expressed at high levels in several cell types present in the skin. Example of a pathogenic variant of ATXN3 are variants with a polyglutamine tract of 50 to 90 residues. An example of a disease associated with a pathogenic variant of ATXN3 is spinocerebellar ataxia 3.

BRCA1 as used herein relates, depending on the context, to the BRCA1 gene or its expression product. BRCA1 is described in detail as gene ID 672 in the NCBI Gene database. The BRCA1 protein has the UniProtKB ID P38398. According to the human protein atlas the BRCA1 protein is expressed at high levels in epidermal cells present in the skin. One example of a pathogenic variant is VAR_007757 where the cysteine at position 61 is changed to a glycine. An example of a disease associated with a pathogenic variant of BRCA1 is breast cancer.

BRCA2 as used herein relates, depending on the context, to the BRCA2 gene or its expression product. BRCA2 is described in detail as gene ID 675 in the NCBI Gene database. The BRCA2 protein has the UniProtKB ID P51587. According to the human protein atlas the BRCA2 protein is expressed at medium levels in Langerhans cells and keratinocytes present in the skin. One example of a pathogenic variant is VAR_063916 where the aspartic acid at position 2723 is changed to a glycine. An example of a disease associated with a pathogenic variant of BRCA2 is breast cancer.

HTT as used herein relates, depending on the context, to the HTT gene or its expression product. HTT is described in detail as gene ID 3064 in the NCBI Gene database. The HTT protein has the UniProtKB ID P42858. According to the human protein atlas the HTT protein is expressed at high levels in the epidermal cells present in the skin. Example of a pathogenic variant of HTT are variants with a polyglutamine tract of 50 to 100 residues. An example of a disease associated with a pathogenic variant of HTT is huntington disease.

FMR1 as used herein relates, depending on the context, to the FMR1gene or its expression product. FMR1 is described in detail as gene ID 2332 in the NCBI Gene database. The FMR1 protein has the UniProtKB ID Q06787. According to the human protein atlas the FMR1 protein is expressed at medium levels in several cell types present in the skin, such as epidermal cells, fibroblasts and keratinocytes. Example of a pathogenic variant of FMR1 is VAR_005234 where the isoleucine at position 304 is changed to an asparagin. An example of a disease associated with a pathogenic variant of FMR1 is fragile X syndrome.

TP53 as used herein relates, depending on the context, to the TP53 gene or its expression product. TP53is described in detail as gene ID 7157 in the NCBI Gene database. The TP53 protein has the UniProtKB ID P04637. According to the human protein atlas the TP53 protein is expressed at low levels in several cell types present in the skin, such as epidermal cells and melanocytes. Example of a pathogenic variant of TP53 is VAR_005995 where the arginine at position 273 is changed to a leucine. An example of a disease associated with a pathogenic variant of TP53 is Li-Fraumeni syndrome.

SERPINA1 as used herein relates, depending on the context, to the SERPINA1 gene or its expression product. SERPINA1 is described in detail as gene ID 5265 in the NCBI Gene database. The SERPINA1 protein has the UniProtKB ID P01009. Example of a pathogenic variant of SERPINA1 is VAR_007005 where the methionin at position 382 is changed to an arginine. An example of a disease associated with a pathogenic variant of SERPINA1 is Alpha-1-antitrypsin deficiency.

GALT as used herein relates, depending on the context, to the GALT gene or its expression product. GALT is described in detail as gene ID 2592 in the NCBI Gene database. The GALT protein has the UniProtKB ID P07902. According to the human protein atlas the GALT protein is expressed at medium levels in several cell types present in the skin, such as endothelial cells, and at high levels in lymphocytes. Example of a pathogenic variant of GALT is VAR_068538 where the valine at position 168 is changed to a leucine. An example of a disease associated with a pathogenic variant of GALT is galactosemia.

HFE as used herein relates, depending on the context, to the HFE gene or its expression product. HFE is described in detail as gene ID 3077 in the NCBI Gene database. The HFE protein has the UniProtKB ID Q30201. According to the human protein atlas the HFE protein is expressed at low levels in several cell types present in the skin, such as epidermal cells and keratinocytes. Example of a pathogenic variant of HFE is VAR_004398 where the cysteine at position 282 is changed to a tyrosine. An example of a disease associated with a pathogenic variant of HFE is hemochromatosis.

ATP7B as used herein relates, depending on the context, to the ATP7B gene or its expression product. ATP7B is described in detail as gene ID 540 in the NCBI Gene database. The ATP7B protein has the UniProtKB ID P35670. According to the human protein atlas the ATP7B protein is expressed at medium levels in epidermal cells. Example of a pathogenic variant of ATP7B is VAR_000714 where the methionine at position 645 is changed to an arginine. This nucleotide change also leads to exon skiping and to the production of a truncated protein. An example of a disease associated with a pathogenic variant of ATP7B is Wilson disease.

PKD1 as used herein relates, depending on the context, to the PKD1 gene or its expression product. PKD1 is described in detail as gene ID 5310 in the NCBI Gene database. The PKD1 protein has the UniProtKB ID P98161. According to the human protein atlas the PKD1 protein is expressed at medium levels in cell types present in the skin such as in Langerhans cells. Example of a pathogenic variant of PKD1 is VAR_011053 where the glutamic acid at position 2771 is changed to a lysine. This variant does not undergo autoproteolytic cleavage. An example of a disease associated with a pathogenic variant of PKD1 is polycystic kidney disease.

PKD2 as used herein relates, depending on the context, to the PKD2 gene or its expression product. PKD2 is described in detail as gene ID 5311 in the NCBI Gene database. The PKD2 protein has the UniProtKB ID Q13563. According to the human protein atlas the PKD2 protein is expressed at medium levels in keratinocytes. Example of a pathogenic variant of PKD2 is VAR_058827 where the aspartic acid at position 511 is changed to a valine. An example of a disease associated with a pathogenic variant of PKD2 is polycystic kidney disease.

NF1 as used herein relates, depending on the context, to the NF1 gene or its expression product. NF1 is described in detail as gene ID 4763 in the NCBI Gene database. The NF1 protein has the UniProtKB ID P21359. According to the human protein atlas the NF1 protein is expressed at high levels in cell types present in the skin such as in fibroblasts. Example of a pathogenic variant of NF1 is VAR_010995 where the arginine at position 1276 is changed to a proline. An example of a disease associated with a pathogenic variant of NF1 is neurofibromatosis.

A "genetic disorder" as used herein is a disorder caused in whole or in part by a change in the DNA sequence away from the normal sequence. Genetic disorders can be caused by a mutation in one gene (monogenic disorder), but also include, for example, multifactorial inheritance disorders and combinations of gene mutations and environmental factors. One prominent example of a genetic disorder is sickle cell disease.

A "monogenic disease" as used herein is a disease for which the clinical features are caused by a mutation impacting a single gene. There a roughly 7000 known human disorders that exhibit Mendelian inheritance patterns. Table 1 of Apgar and Sanders (12) provides an exemplary list of the more common monogenic disorders and their associated genes. This table is herein incorporated by reference.

A "de novo mutation" as used herein is a mutation or alteration of the genome in an individual that was not inherited from its parents. De novo mutations can occur, for example, during gamete formation, giving rise to a child having a genetic defect that is not present in either one of its parents. De novo mutations can also occur during embryonic or fetal development or even postnatal during life.

A "mosaic disorder" as used herein is a disorder caused by a pathogenic mutation or alteration of the genome that is not present in all cells of an individual. Thus, mosaicism occurs when an individual has two or more genetically different sets of cells in his or her body. If one of these sets of cells comprises a pathogenic protein variant, a "mosaic disorder" can occur. Females are an example of genetic mosaics since either one or the other X-chromosome of a female is selectively inactivated in any given cell.

Polyglutamine (polyQ) diseases are a group of neurodegenerative disorders caused by expansion of polyglutamine repeats in their associated pathogenic protein variants. In addition to neurodegeneration, polyQ-expanded proteins can cause a wide array of abnormalities in peripheral tissues. The peripheral effects of polyQ disease proteins include muscle wasting and reduced muscle strength in patients. Examples of polyQ diseases are spinal and bulbar muscular atrophy (SBMA), Huntington's disease (HD), dentatorubral-pallidoluysian atrophy (DRPLA), and spinocerebellar ataxia type 17 (SCA17).

A neurodegenerative disease is caused by the progressive loss of structure or function of neurons, in a process known as neurodegeneration. Such neuronal damage may ultimately involve cell death. Non-limiting examples of neurodegenerative diseases are amyotrophic lateral sclerosis, multiple sclerosis, Parkinson's disease, Alzheimer's disease, Huntington's disease, multiple system atrophy, tauopathies, and prion diseases.

An inborn error of immunity (IEI) is a genetic mutation that results in an increased susceptibility to infectious disease, autoinflammatory disease, allergy, or autoimmunity. Inborn errors include, but are not limited to, primary immunodeficiencies.

A primary immunodeficiency as used herein is a disorder resulting from a functional defects of the adaptive and/or innate immune system. Non-limiting examples of primary immunodeficiencies are T-cell deficiencies, B-cell deficiencies, phagocyte defects, complement defects and combined immunodeficiencies. Examples are an IgG subclass deficiency, selective IgA deficiency, IRAK4 deficiency, JAK3 deficiency, to name but a few.

"Topical" as used herein means the application of a drug or a diagnostic agent, for example IgE or an IgE-containing composition, to an external body surface, in particular to the skin.

A "companion diagnostic" as used herein means a product that is used for determining and/or verifying the safe and effective use of a related medicament in order to identify patients before and/or during treatment who are most likely to benefit from the associated medicinal product, and/ or to identify patients before and/or during treatment who are likely to be at increased risk of serious adverse reactions as a result of treatment with the associated medicament.

A "unit dose" as used herein is the amount of a medication administered to a patient in a single dose.

Medical or physiological terms, like "erythema", as used herein have the meaning as known to the experienced general practitioner or medical doctor; unless specified otherwise. In cases of doubt, the English language version of the book "Pschyrembel" can be used to define medical or physiological terms.

"A concentration sufficient to cause erythema" as used herein is a concentration of IgE which causes erythema in individuals who express the protein which is specifically bound by the IgE in the skin, e.g. the concentration which causes erythema in at least 70% of individuals, preferably at least 85% of individuals, from a randomly selected cohort of healthy individuals representative for the worldwide age distribution at the priority date (in the case where IgE binds specifically to the non-pathogenic version of said protein).

A "lack of the expected erythema" as used herein means that there is no sign of the development of an erythema at the site of administration within 24 hours after topical administration of the IgE.

As used herein, the term "subject" or "individual" as used herein denotes a human being.

In the context of the invention, the term "treating" or "treatment", as used herein, means reversing, alleviating, inhibiting the progress of, or preventing the disorder, disease or condition to which such term applies, or one or more symptoms of such disease or condition. Preferably it means reversing, alleviating, inhibiting the progress of, or preventing the disease or condition.

The term "diagnosing" as used herein refers to assessing the probability according to which a subject expresses a pathogenic variant of a protein as referred to in this specification. As will be understood by those skilled in the art, such an assessment is usually not intended to be correct for 100% of the subjects to be diagnosed. The term, however, requires that a statistically significant portion of subjects can be correctly diagnosed to be expressing said pathogenic variant of a protein. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983 . Preferred confidence intervals are at least 90%, at least 95%, at least 97%, at least 98% or at least 99 %. The p-values are, preferably, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

"Non-invasive" as used herein refers to a diagnostic method which does not comprise a step, where the dermis and/or an endothelium is breached. A skin-prick test, similar to the allergy tests which are in current use, is considered non-invasive. For example, a diagnostic method which relies on the taking of blood with a needle would include at least one step wherein an endothelium is breached and is thus an invasive diagnostic method as used herein.

As used herein, an IgE "binds specifically to" or "specifically binds to" a pathogenic variant of a protein if such IgE is able to discriminate between said pathogenic variant of a protein and the non-pathogenic reference for said protein, since binding specificity is not an absolute, but a relative property. For example, an ELISA assay can be carried out with the IgE and wells coated with the pathogenic variant and wells coated with the non-pathogenic reference. The reaction in wells is then scored depending on the type of output signal that the linked enzyme in the enzyme linked immunoassay provides. In an assay with a positive signal rationale (i.e. the higher the binding the higher the signal), the difference between the signal for the pathogenic variant of the protein of interest and the non-pathogenic reference for said protein should be at least 5-fold, such as at least 10-fold, for example at least 50-fold.

As used herein, the term "affinity" refers to the strength of interaction between the IgE and its antigen of interest. Typically, the binding region of the IgE interacts through weak non-covalent forces with its antigen. For example, for an IgE antibody, the interaction with the antigen is typically mediated by the CDRs.

The term "Kd", as used herein, refers to the dissociation constant, which is obtained from the ratio of Kon to Koff and is expressed as a molar concentration (M). Kd values for antigen binding moieties like e.g. monoclonal antibodies can be determined using methods well established in the art. Methods for determining the Kd of an antigen binding moiety like e.g. a monoclonal antibody are SET (soluble equilibrium titration) or surface plasmon resonance using a biosensor system such as a Biacore^{®} system. In the present disclosure an antibody specific to the target antigen typically has a dissociation rate constant (KD ) (koff/kon) of less than 100nM, such as less than 10nM, preferably less than 1000pM, and in particular less than 100pM when measured in 1xPBS (pH7.3-7.5) at 25 degrees Celsius.

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about." It also is to be understood, although not always explicitly stated, that the reagents described herein are merely examples and that equivalents of such are known in the art.

It is to be understood that this invention is not limited to the particular materials and methods described herein. It is also to be understood that the terminology used herein is for the purpose of describing particular embodiments and is not intended to limit the scope of the present invention, which will be limited only by the appended claims. As used herein, the singular forms "a", "an", and "the" include plural reference unless the context clearly indicates otherwise.

Unless defined otherwise, all technical and scientific terms used herein have the same meanings as commonly understood by one of ordinary skill in the art to which this invention belongs. The following references provide one of skill with a general definition of many of the terms used in this invention - unless defined otherwise herein - and ranked in increasing order of priority: Singleton et al., Dictionary of Microbiology and Molecular Biology (3rd ed. 2006); The Glossary of Genomics Terms (JAMA. 2013; 309(14): 1533-1535), Janeway's Immunobiology, 9th edition and "Practical Flow Cytometry", 4th edition by H.M. Shapiro.

All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

The invention further relates to the following embodiments
1. IgE for use in a diagnostic method.
2. IgE for use according to item 1, wherein the diagnostic method is practiced on the human or animal body.
3. IgE for use according to items 1 to 2, wherein the diagnostic method is practiced on the skin, in particular on the human skin.
4. IgE for use according to items 1 to 3, wherein the diagnostic method comprises a skin-prick test.
5. IgE for use in a diagnostic method according to any one of items 1 to 4, wherein the IgE binds a pathogenic variant of a protein, but not the non-pathogenic version of said protein.
6. IgE for use in a diagnostic method according to any one of items 1 to 4, wherein the IgE binds the non-pathogenic version of a protein, but not a pathogenic variant of said protein.
7. IgE for use in a diagnostic method according to any one of items 5 to 6, wherein the pathogenic variant of said protein is an extended protein.
8. IgE for use in a diagnostic method according to any one of items 5 to 6, wherein the pathogenic variant of said protein is a mutated protein.
9. IgE for use in a diagnostic method according to any one of items 5 to 6, wherein the pathogenic variant of said protein differs from the non-pathogenic version in a covalent protein modification.
10. IgE for use in a diagnostic method according to item 9, wherein the covalent protein modification is selected from phosphorylation, glycosylation, lipidation, ubiquitination, disulfide formation, cleavage of a peptide bond and carbonylation.
11. IgE for use in a diagnostic method according to any one of items 1 to 10, wherein the IgE binds the protein encoded by the SMN1 gene.
12. IgE for use in a diagnostic method according to any one of items 1 to 10, wherein the IgE binds the protein encoded by the DMD gene.
13. IgE for use in a diagnostic method according to any one of items 1 to 10, wherein the IgE binds the protein encoded by the LDLR gene.
14. IgE for use in a diagnostic method according to any one of items 1 to 10, wherein the IgE binds the protein encoded by the BRCA1 gene.
15. IgE for use in a diagnostic method according to any one of items 1 to 10, wherein the IgE binds the protein encoded by the BRCA2 gene.
16. IgE for use in a diagnostic method according to any one of items 1 to 10, wherein the IgE binds the protein encoded by the HTT gene.
17. IgE for use in a diagnostic method according to any one of items 1 to 10, wherein the IgE binds the protein encoded by the FMR1 gene.
18. IgE for use in a diagnostic method according to any one of items 1 to 10, wherein the IgE binds the protein encoded by the ATXN1 gene.
19. IgE for use in a diagnostic method according to any one of items 1 to 10, wherein the IgE binds the protein encoded by the ATXN2 gene.
20. IgE for use in a diagnostic method according to any one of items 1 to 10, wherein the IgE binds the protein encoded by the ATXN3 gene.
21. IgE for use in a diagnostic method according to any one of items 1 to 10, wherein the IgE binds the protein encoded by the TP53 gene.
22. IgE for use in a diagnostic method according to any one of items 1 to 10, wherein the IgE binds the protein encoded by the SERPINA1 gene.
23. IgE for use in a diagnostic method according to any one of items 1 to 10, wherein the IgE binds the protein encoded by the GALT gene.
24. IgE for use in a diagnostic method according to any one of items 1 to 10, wherein the IgE binds the protein encoded by the IGHA1 gene.
25. IgE for use in a diagnostic method according to any one of items 1 to 10, wherein the IgE binds the protein encoded by the HFE gene.
26. IgE for use in a diagnostic method according to any one of items 1 to 10, wherein the IgE binds the protein encoded by the ATP7B gene.
27. IgE for use in a diagnostic method according to any one of items 1 to 10, wherein the IgE binds the protein encoded by the PKD1 gene.
28. IgE for use in a diagnostic method according to any one of items 1 to 10, wherein the IgE binds the protein encoded by the PKD2 gene.
29. IgE for use in a diagnostic method according to any one of items 1 to 10, wherein the IgE binds the protein encoded by the NF1 gene.
30. IgE for use in a diagnostic method according to any one of items 1 to 10, wherein the IgE binds the protein encoded by the CASP8 gene.
31. IgE for use in a diagnostic method according to any one of items 1 to 10, wherein the IgE binds the protein encoded by the CARD9 gene.
32. IgE for use in a diagnostic method according to any one of items 1 to 10, wherein the IgE binds the protein encoded by the CTLA4 gene.
33. IgE for use in a diagnostic method according to any one of items 1 to 10, wherein the IgE binds polyglutamine.
34. IgE for use in a method of diagnosing a monogenic disorder.
35. IgE for use in a method of diagnosing a polyglutamine disease.
36. IgE for use in a method of diagnosing a neurodegenerative disease.
37. IgE for use in a method of diagnosing an inborn error of immunity.
38. IgE for use according to item 37, wherein the inborn error of immunity is a primary immunodeficiency.
39. IgE for use according to any one of items 1 to 38, wherein the protein to be detected is expressed in at least one cell type present in the skin, e.g., at at least a low level.
40. IgE for use according to any one of items 1 to 38, wherein the protein to be detected is expressed in at least one cell type present in the skin at at least a medium level.
41. IgE for use according to any one of items 1 to 38, wherein the protein to be detected is expressed in at least one cell type present in the skin at at least a high level.
42. IgE for use according to any one of items 39 to 41, wherein the cell type present in the skin is an epidermal cell.
43. IgE for use according to any one of items 39 to 41, wherein the cell type present in the skin is a fibroblast.
44. IgE for use according to any one of items 39 to 41, wherein the cell type present in the skin is a lymphocyte.
45. IgE for use according to any one of items 39 to 41, wherein the cell type present in the skin is a Langerhans cell.
46. IgE for use according to any one of items 39 to 41, wherein the cell type present in the skin is a keratinocyte.
47. IgE for use according to any one of items 39 to 41, wherein the cell type present in the skin is a melanocyte.
48. Use of an aqueous solution comprising IgE in a skin-prick test.
49. Topical use of IgE for the detection of a protein.
50. Use of IgE as a companion diagnostic for a medicament.
51. Use according to item 50, wherein the medicament is for the treatment of a genetic disorder.
52. Use according to item 51, wherein the genetic disorder is monogenic.
53. Use according to item 52, wherein the genetic disorder is autosomal dominant, autosomal recessive or X-linked.
54. An aqueous solution comprising IgE for diagnostic use according to any one of items 1 to 53
55. A diagnostic method for detecting the presence of a pathogenic variant of a protein in the skin wherein,
   a) IgE that is capable of binding a pathogenic variant of said protein, but not the non-pathogenic version of said protein, is applied to the skin;
   b) the skin is pricked at the site where IgE was applied to, allowing IgE to enter the skin; and
   c) the development of an allergic response, such as an erythema, is indicative of the presence of a pathogenic variant of said protein.
56. A diagnostic method for detecting the absence of a protein in the skin wherein,
   a) IgE that is capable of binding the non-pathogenic version of said protein is applied to the skin;
   b) the skin is pricked at the site where IgE was applied to, allowing IgE to enter the skin; and
   c) the absence of the development of an allergic response, such as an erythema, is indicative of the absence of said protein.
57. IgE as a companion diagnostic for a medicament, wherein the medicament is for the treatment of a genetic disorder and wherein the genetic disorder is autosomal dominant, autosomal recessive or X-linked.
58. A method of treating a subject having a genetic disorder, such as an autosomal dominant, autosomal recessive or X-linked disorder, comprising administering an effective amount of a medicament effective for the treatment of said disorder to said subject, wherein the disorder has been diagnosed using a method of diagnosis of the invention.
59. A method of treating a subject having spinal muscular atrophy, comprising administering an effective amount of a survival of motor neuron 2-directed RNA splicing modifier or comprising administering an effective amount of a gene therapy, such as an adeno-associated virus (AAV) vector-based gene therapy, to said subject, wherein SMA has been diagnosed using a method of diagnosis of the invention.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Handbook of Experimental Immunology" (Weir, 1997); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques may be considered in making and practicing the invention.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### Discussion

In a classical skin-prick test small amounts of allergen are introduced into the epidermis and non-vascular superficial dermis. When IgE specific for the introduced allergen is present and bound to cutaneous mast cells, the allergen can interact with it and trigger the release of histamine and other mediators, leading to a visible "wheal-and-flare" reaction, typically peaking after about 15 minutes.

The invention here suggests that externally added IgE can also trigger the allergic reaction when it binds to its specific target molecule. This means that IgE that is pricked into the skin will trigger an allergic skin reaction dependent on the presence of its target protein in the skin. Thus, an IgE that is specific for a pathogenic protein variant will trigger a skin reaction if said pathogenic protein variant is present in the skin.

Abbreviations:
TLR: toll-like receptor
EMA: European Medicines Agency
FDA: US Food and Drug Administration
RNA: ribonucleic acid

## Claims

1. IgE for use in a diagnostic method practiced on the human or animal body.

2. IgE for use according to claim 1, wherein the method comprises administering IgE to a subject in a skin-prick test.

3. IgE for use according to any one of claims 1 to 2, wherein the IgE is capable of binding specifically a pathogenic variant of a protein, but not a non-pathogenic version of said protein.

4. IgE for use according to claim 3, wherein the method comprises
a) applying IgE that is capable of binding the pathogenic variant of said protein, but not a non-pathogenic version of said protein, to a site of the skin; and
b) pricking the skin at said site ;
c) wherein the development of an erythema is indicative of the presence of the pathogenic variant of said protein.

5. IgE for use according to any one of claims 1 to 2, wherein the IgE is capable of binding specifically a non-pathogenic version of a protein, but not a pathogenic variant of said protein.

6. IgE for use according to any one of claims 1 to 5 in the diagnosis of a genetic disorder, optionally, a monogenic disorder.

7. IgE for use according to any one of claims 1 to 6, wherein IgE is capable of binding a protein encoded by any one gene selected from CFTR, SMN1, DMD, LDLR, ATXN1, ATXN2, ATXN3, BRCA1, BRCA2, HTT, FMR1, TP53, SERPINA1, GALT, IGHA1, CARD9, CASP8, CTLA4 or wherein IgE is capable of binding polyglutamine.

8. IgE for use according to any one of claims 1 to 7, wherein the IgE is a companion diagnostic for a medicament.

9. IgE for use according to claim 9, wherein the medicament is for the treatment of a genetic disorder, optionally, a monogeneic disorder.

10. IgE for use according to any of claims 6 or 9, wherein the monogenic disorder is cystic fibrosis, a neurodegenerative disorder or a primary immunodeficiency.

11. A diagnostic method for detecting the presence of a pathogenic variant of a protein in skin, comprising
a) applying IgE that is capable of binding the pathogenic variant of said protein, but not a non-pathogenic version of said protein, to a site of the skin; and
b) pricking the skin at said site thereby allowing IgE to enter the skin ;
c) wherein the development of an erythema is indicative of the presence of the pathogenic variant of said protein.

12. A diagnostic method for detecting the presence of a non-pathogenic variant of a protein in skin, comprising
a) applying IgE that is capable of binding the non-pathogenic version of said protein, but not a pathogenic variant of said protein, to a site of the skin; and
b) pricking the skin at said site, thereby allowing IgE to enter the skin;
c) wherein the development of an erythema is indicative of the presence of the non-pathogenic variant of said protein.
